# EUROPEAN PATENT SPECIFICATION

(11) **EP 3 174 591 B1**
(45) Date of publication and mention of the grant of the patent: **15.11.2023**
(21) Application number: 15827400.1
(22) Date of filing: 27.07.2015
(51) Int. Cl.: A61B 17/22, A61M 25/00

(54) **TRANSCAROTID NEUROVASCULAR CATHETER**
NEUROVASKULÄRER TRANSKAROTISKATHETER
CATHÉTER NEUROVASCULAIRE TRANSCAROTIDIEN

(30) Priority: 28.07.2014 US 201462029799 P; 04.09.2014 US 201462046112 P; 04.11.2014 US 201462075169 P; 04.11.2014 US 201462075101 P; 21.11.2014 US 201462083128 P
(43) Date of publication of application: 07.06.2017
(73) Proprietor: Silk Road Medical, Inc., Sunnyvale, California 94089 (US)
(72) Inventor: GARRISON, Michi E., Sunnyvale, CA 94085 (US)
(74) Representative: Slingsby Partners LLP
(86) International application number: PCT/US2015/042180
(87) International publication number: WO 2016/018781

(56) References cited:
- US-A1- 2002 156 460
- US-A1- 2004 019 322
- US-A1- 2006 089 618
- US-A1- 2009 030 400
- US-A1- 2011 112 567
- US-A1- 2011 238 041
- US-A1- 2011 238 041
- US-A1- 2013 281 788

## Description

### BACKGROUND

Intravascular catheters are used to access target vascular regions from remote vascular access sites to perform a procedure. The design, materials, and construction of particular catheters are primarily directed to allow the catheter to reach the target vascular anatomy while not causing vessel trauma, as well as to perform the catheter's intended function upon the catheter reaching the target anatomy. The catheter often has multiple requirements that may conflict with one another. Consequently, a strong design optimally balances the goals of these requirements.

Many catheters are single lumen catheters wherein the lumen acts as a channel for the delivery of radiopaque or therapeutic agents or for other interventional devices into the blood vessel, and/or for aspiration of blood, thrombus, or other occlusive material out of the blood vessel. Such catheters have physical properties that allow them to be advanced through a vessel access site from a proximal end into vascular anatomy which is often very curved, delicate, tortuous, and remote from the blood vessel access site. These catheters are also designed to be used with adjunctive devices such as guide wires and sometimes smaller catheters positioned in the inner lumen, and to be directed to the target anatomy through vascular access sheaths, guide catheters and sometimes sub-selective guide catheters (i.e. catheters that are specifically designed to go to more distal locations than typical guide catheters). In other words, it is often not a single catheter but a system of catheters, guide wires, guide catheters, and sheaths that allows the user to adequately perform an intended procedure.

Interventions in the cerebral vasculature often have special access challenges. Most neurointerventional procedures use a transfemoral access to the carotid or vertebral artery and thence to the target cerebral artery. However, this access route is often tortuous and may contain stenosic plaque material in the aortic arch and carotid and brachiocephalic vessel origins, presenting a risk of embolic complications during the access portion of the procedure. In addition, the cerebral vessels are usually more delicate and prone to perforation than coronary or other peripheral vasculature. In recent years, interventional devices such as wires, guide catheters, stents and balloon catheters, have all been scaled down and been made more flexible to better perform in the neurovascular anatomy. However, many neurointerventional procedures remain either more difficult or impossible because of device access challenges. In some instances, a desired access site is the carotid artery. Procedures in the intracranial and cerebral arteries are much closer to this access site than a femoral artery access site. Importantly, the risk of embolic complications while navigating the aortic arch and proximal carotid and brachiocephalic arteries are avoided. However, because most catheters used in interventional procedures are designed for a femoral access site, current devices are not ideal for the alternate carotid access sites, both in length and mechanical properties. This makes the procedure more cumbersome and in some cases more risky if using devices designed for femoral access in a carotid access procedure.

U.S. Patent Number 5,496,294 (the `294 patent) describes a single lumen, three-layer catheter design, including (1) an inner Polytetrafluoroethylene (PTFE) liner to provide a low-friction inner surface; (2) a reinforcement layer formed of a metal coil wire or coil ribbon; and (3) an outer jacket layer. Typically, the three layers are laminated together using heat and external pressure such as with heat shrink tubing. The catheter has multiple sections of varying stiffness such that flexibility increases moving towards the distal end of the catheter. This variation in flexibility may be accomplished by varying the durometer of the outer jacket layer along the length of the catheter. Another method to vary flexibility is by varying the reinforcement structure and/or material along the length of the catheter.

One limitation in the `294 patent and in other existing neurovascular catheter technology is that the devices are designed for a femoral access approach to the cerebral arteries. The pathway from the femoral artery to the common carotid artery and thence to the internal carotid artery is both long and comprises several back and forth bends. The dimensions provided in the `294 patent are consistent with this design goal. However, catheters designed to navigate this route have lengths and flexibility transitions that would not be appropriate for a transcarotid access, and would in fact detract from performance of a transcarotid catheter. For example the flexible sections must be at least 40 cm of gradually increasing stiffness from the distal end to a proximal-most stiff section, to be able to navigate both the internal carotid artery curvature and the bends required to go from the aortic arch into the common and then the internal carotid artery.

Another disadvantage to the catheter construction described in the `294 patent is the catheter's limited ability to have continuous, smooth transitions in flexibility moving along its length. There are discreet differences in flexibility on a catheter where one jacket material abuts another, or when one reinforcement structures abuts another reinforcement structure. In addition, the tri-layer catheter construction of the `294 patent has limitations on the wall-thickness due to the need to be able to handle and assemble the three layers of the catheter during manufacture. In addition, the catheter construction makes it difficult to have a relatively large inner lumen diameter while maintaining properties of flexibility and/or kink resistance to very sharp bends in the blood vessel. As a general rule, the larger diameter catheters also tend to be stiffer in order to remain kink resistant.

US 2011/238041 A1 discloses an interventional catheter for treating an artery, comprising an elongated body sized and shaped to be transcervically introduced into a common carotid artery at an access location in the groin.

### SUMMARY

There is a need for a catheter with dimensions and mechanical properties which have been optimized to access the cerebral vessels from a carotid artery access site. There is also a need to for a catheter that has gradual, smooth transitions from a first flexibility to at least a second, different flexibility moving along the length of the catheter. There is also a need for a catheter that has a relatively large inner diameter compared to prior art catheters, and yet is able to maintain physical properties such as thin wall thickness, kink resistance and flexibility.

The invention is defined in claim 1. Further embodiments of the invention are defined in the dependent claims. Methods are not claimed.

Disclosed is an intravascular catheter that that has been optimized for accessing anterior cerebral vessels from a carotid artery access site.

Disclosed also is a catheter which includes variations in flexibility or stiffness moving along at the entire length of catheter or a portion of the length of the catheter. Advantageously, the change in flexibility of the catheter is represented by smooth, rather than sudden, changes in flexibility. In other words, the flexibility of the catheter transitions gradually moving along its length without any sudden or discrete variations in flexibility from one section of the catheter to an adjacent section of the catheter. As described in more detail below, the catheter can be particularly sized and shaped according to how the catheter will be used and in what particular section of the vascular anatomy the catheter will be used.

Also disclosed is an interventional catheter for treating an artery, comprising: an elongated body sized and shaped to be transcervically introduced into a common carotid artery at an access location in the neck, the elongated body sized and shaped to be navigated distally to an intracranial artery through the common carotid artery via the access location in the neck; an internal lumen in the elongated body, the internal lumen forming a proximal opening in a proximal region of the elongated body and a distal opening in a distal region of the elongated body; wherein the elongated body has a proximal most section and a distal most section wherein the proximal most section is a stiffest portion of the elongated body, and wherein the elongated body has an overall length and a distal most section length such that the distal most section can be positioned in an intracranial artery and while simultaneously at least a portion of the proximal most section is positioned in the common carotid artery during use.

Other features and advantages should be apparent from the following description of various embodiments, which illustrate, by way of example, the principles of the disclosure.

### BRIEF DESCRIPTION OF THE DRAWINGS

Figure 1A shows a schematic view of an exemplary catheter.
Figure 1B shows a schematic view of an alternate embodiment of a catheter.
Figures 2-5 show examples of catheters having angled distal tips or distal edges.
Figure 6 illustrates an embodiment of a catheter with a tapered co-axial inner member.
Figure 7 illustrates another embodiment of a catheter with a tapered co-axial inner member.

### DETAILED DESCRIPTION

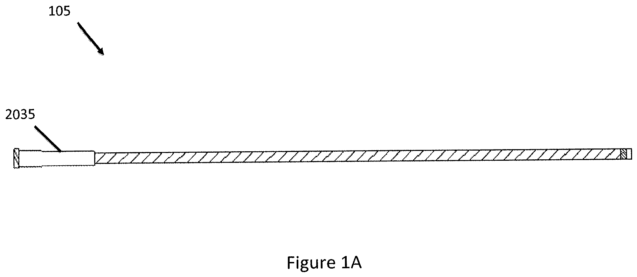

Figure 1A shows a schematic view of an exemplary catheter 105. The catheter 105 is an elongated body that has an external dimension that is sized and shaped for insertion into a blood vessel. In an embodiment, the catheter 105 is sized and shaped for insertion into an access sheath of a carotid artery access system such as described in U.S Patent Application Serial No. 12/834,869 entitled SYSTEMS AND METHODS FOR TREATING A CAROTID ARTERY. U.S Patent Application Serial No. 14/537,316 entitled METHODS AND DEVICES FOR TRANSCAROTID ACCESS and filed November 10, 2014 is also cited.

The proximal region of the catheter 105 may have one or more mechanical or electro-mechanical control mechanisms for controlling different components on or near a distal end of the catheter 105. For example, the control mechanism(s) can be used to control inflation of a balloon or balloons, advancement/deployment of a system component (such as a stent), flushing or aspirating a fluid through the catheter, and combinations thereof.

With reference again to Figure 1A, the catheter 105 is configured to be inserted through an access sheath in the carotid artery and navigated distally to the distal ICA or cerebral vessels. A proximal port 2035 with a hemostasis valve may be situated on the proximal end of catheter 105, to allow introduction of devices such as a microcatheter, guide wire, stent delivery device, aneurysm coil delivery device, or thrombectomy device while preventing or minimizing blood loss during the procedure. The hemostasis valve may be integral to the catheter proximal adaptor, or may be removably attached to the proximal end of the catheter via a proximal connector. In an embodiment, this valve is an adjustable-opening valve such as a Tuohy-Borst or rotating hemostasis valve (RHV). In another embodiment, the valve is a passive seal hemostasis valve.

The catheter 105 may be made with a two or more layer construction. In an embodiment, the catheter has a PTFE inner liner, an outer jacket layer, and at least a portion of the catheter has a reinforcement structure, such as a tubular structure formed of, for example, a wound coil, braid or cut hyptotube. In addition, the catheter may have a radiopaque marker at the distal tip to facilitate placement of the device using fluoroscopy.

The catheter 105 has an insertable portion (or working length) that is sized to be inserted through an access sheath in the carotid artery and passed through an arterial pathway (through the artery) to the distal ICA or cerebral vessels. In an embodiment adapted to be used with an access sheath of total length including the sheath hemostasis valve of about 15 to 20 cm, the catheter 105 has a working length ranging from 40 to 70 cm. The working length (or insertable portion) of the catheter is the portion of the catheter that is sized and shaped to be inserted into the artery and wherein at least a portion of the working length is actually inserted into the artery during a procedure. In an embodiment, the catheter has a working length of less than 70 cm, less than 60 cm, or less than 50 cm. A similar catheter designed for a transfemoral access site may have a working length of between 100 and 130 cm. Alternately, the length of catheter can be defined relative to the location of the access site and the target cerebral artery site. In an embodiment, the catheter is configured to be introduced into the artery at a location in the artery that is less than 40 cm, less than 30 cm, or less than 20 cm from the location of the target site as measured through the arterial pathway. The distance may further be defined by a ratio of working length to the distance between the location where the catheter enters the arteriotomy and the target site. In an embodiment, this ratio is less than 2x. In an embodiment, the working length of the device may have a hydrophilic coating to improve the ease of advancement of the device through the vasculature. In an embodiment, at least 40% of the working length of the catheter is coated with a hydrophilic material. In other embodiments, at least 50% or at least 60% of the working length of the catheter is coated with a hydrophilic material. In an embodiment, the elongated body has an overall length and a distal most section or portion length such that the distal most section can be positioned in an intracranial artery and at least a portion of the proximal most section 115 (Figure 1A) is positioned in the common carotid artery during use while transcervically inserted into the common carotid artery.

In an embodiment, the distal-most section 111 (Figure 1A) is constructed to be more flexible than the proximal portion, with one or more flexible sections, to successfully navigate the internal carotid artery curvature to reach target sites in the distal (internal carotid artery) ICA or cerebral arteries. The shaft may have a transition, or intermediate, section 113 of one or more increasingly stiff sections towards the more proximal section of the shaft, with the proximal most portion having the stiffest shaft section. Alternately, the transition section is a section of continuously variable stiffness from the distal section stiffness to the proximal section stiffness. In an embodiment, the distal most flexible section is between 5 and 15 cm. In another embodiment, the distal most flexible section is between 3 and 10 cm. In another embodiment, the distal section is between 2 and 7 cm. In an embodiment, the transition section is between 5 and 15 cm. In another embodiment, the transition section is between 5 and 10 cm. In another embodiment, the transition section is between 4 and 8 cm. In all these embodiments, the proximal-most stiff section takes up the remainder of the working length. In an embodiment where the catheter has a working length of 40 cm, the proximal-most stiff section is in a range 10 to 30 cm. In an embodiment where the catheter has a working length of 70 cm, the proximal-most stiff section is in a range from 40 to 60 cm. In an embodiment, the stiffest portion of the catheter is the proximal most portion of the catheter. The catheter can have a length such that, when inserted into the common carotid artery via a transcarotid entryway into the artery, the stiffest section of the catheter is located at least partially within the common carotid artery or can be positioned at least two centimeters into the common carotid artery. In an embodiment, the catheter has a length such that at least portion of the proximal most section is positioned in the common carotid artery when the distal most section is in an intracranial artery during use. The relative lengths of distal most section, transition section, and proximal most sections are not necessarily shown to scale in Figure 1A.

Alternately, the flexible distal section and transition section may be described as a portion of the overall catheter working length. In an embodiment, the flexible distal most section is between 3 to 15% of the length of the working length of the catheter. In another embodiment, the flexible distal most section is between 4 and 25% of the length of the working length of the catheter. Similarly, in an embodiment, the transition section is between 7 and 35% of the length of the working length of the catheter. In another embodiment, the transition section is between 6 and 20% of the working length of the catheter.

In an embodiment, the flexibility of the distal most section is in the range 3 to 10 N-mm² and the flexibility of the proximal post section is in the range 100 to 500 N-mm², with the flexibility/flexibilities of the transition section falling between these two values.

As noted above, the catheter may have sections with discreet and/or continuously variable stiffness shaft. The sections of varying flexibility may be achieved by multiple methods. For example, the outer jacket layer may be composed of discreet sections of polymer with different durometers, composition, and/or thickness. In another embodiment, the outer layer has one or more sections of continuously variable outer layer material that varies in flexibility. The catheter may be equipped with the continuously variable outer layer material by dip coating the outer layer rather than laminating a jacket extrusion onto a PTFE-liner and reinforcement assembly of the catheter. The dip coating may be, for example, a polymer solution that polymerizes to create the outer jacket layer of the catheter. The smooth transition from one flexibility (e.g., durometer) to another flexibility along the length of the catheter can be accomplished via dipping the catheter assembly in multiple varying durometer materials whereby the transition from one durometer to another can be accomplished in a graded pattern, for example by dipping from one side of the catheter in one durometer with a tapering off in a transition zone, and dipping from the other side in another durometer with a tapering off in the same transition zone, so there is a gradual transition from one durometer to the other. In this embodiment, the dip coating can create a thinner walled outer jacket than a lamination assembly. In another embodiment, the catheter has an outer jacket layer that is extruded with variable durometer along the length, to provide variable flexibility along the length of the catheter.

In an embodiment, at least a portion of the catheter has a reinforcement structure, such as a tubular structure formed of, for example, a wound coil, braid that is composed of discreet or continuously varying structure to vary the stiffness, for example a variable coil or braid pitch. In an embodiment, the reinforcement structure is a cut hyptotube, with a cut pattern that is graded along the length, for example cut in a spiral pattern with continuously variable pitch or continually variable cut gap, or a repeating cut pattern that allows the tube to flex whereby the repeating pattern has a continuously variable repeat distance or repeat size or both. A cut hypotube-reinforced catheter may also have superior pushability than a coil-reinforced catheter, as it is a structure with potentially greater stability in the axial direction than a wound coil. The material for the reinforcement structure may be stainless steel, for example 304 stainless steel, nitinol, cobalt chromium alloy, or other metal alloy that provides the desired combination of strengths, flexibility, and resistance to crush. In an embodiment, the reinforcement structure comprises multiple materials along the different sections of flexibility

In another embodiment the catheter has a PTFE inner liner with one or more thicknesses along variable sections of flexibility. In an embodiment, in accordance with the present claimed invention, the PTFE inner liner is constructed to be extremely thin, between .0005" and .0010". 1 inch = 2.54 cm.

This embodiment provides the catheter with a high level of flexibility as well as the ability to construct a thinnerwalled catheter. For example, the PTFE liner is constructed by drawing a mandrel through a liquid PTFE liquid solution rather than the conventional method of thin-walled PTFE tubing manufacture, namely extrusion of a PTFE paste which is then dried and sintered to create a PTFE tube. The draw method allows a very thin and controlled wall thickness, such as in the range of .0005" - .0010".

Any one of the aforementioned manufacturing methods may be used in combination to construct the desired flexibility and kink resistance requirement. Current tri-layer catheters have wall thicknesses ranging from .005" to .008". These manufacturing techniques may results in a catheter with better catheter performance at the same wall thickness, or with equal or better catheter performance at lower wall thicknesses for example between .003" to .005".

In an embodiment, the distal flexible section of the catheter may be constructed using one or more of: a dip coated outer layer, an extremely thin drawn PTFE layer, and a cut hypotube reinforcement layer, with a gradual transition from the flexible section to a stiffer proximal section. In an embodiment, the entire catheter is constructed with one or more of these elements

In some instances, there is a need to reach anatomic targets with the largest possible internal lumen size for the catheter. For example the catheter may be used to aspirate an occlusion in the blood vessel. Thus there is a desire to have a very flexible, kink resistant and collapse resistant catheter with a thin wall and large inner diameter. A catheter using the

In some instances, there is a need to reach anatomic targets with the largest possible internal lumen size for the catheter. For example the catheter may be used to aspirate an occlusion in the blood vessel. Thus there is a desire to have a very flexible, kink resistant and collapse resistant catheter with a thin wall and large inner diameter. A catheter using the construction techniques disclosed herein meets these requirements. 1 inch = 2.54 cm. For example, the catheter may have an inner diameter of .068" to .095" and a working length of 40-60 cm. In another embodiment, the catheter may be sized to reach the more distal cerebral arteries, with an inner diameter of .035" to .062" and a working length of 50-70 cm. In an embodiment, the catheter is configured to navigate around a 180° bend around a radius as small as .050" or .100" without kinking, wherein the bends are located within 5 cm, 10 cm, or 15 cm of the arteriotomy measured through the artery. In an embodiment, the catheter can resist collapsing whilst in a tortuous anatomy up to 180° x .050" radius bend without collapsing when connected to a vacuum up to 67.728 kPa (20 inHg). In an embodiment, the catheter can resist collapse in the same conditions when connected to a vacuum up to 84.660 kPa (25 inHg).

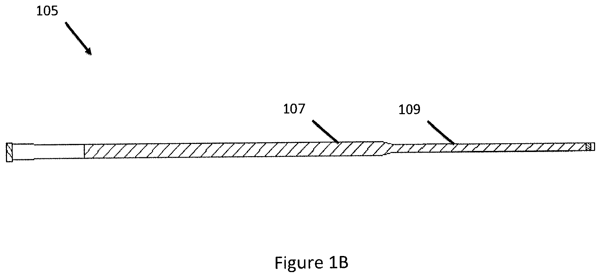

In another embodiment shown in Figure 1B, the inner and outer diameter may be stepped up at a proximal region 107 of the catheter. The step up corresponds to an increase in diameter relative to an adjacent region of the catheter. This embodiment would further optimize the aspiration power of the catheter. For example, the portion of the catheter which is in more proximal, larger vessels during a procedure may have a larger diameter than distal region 109 of the catheter, which can be the distal most region. In this embodiment, the catheter may have a diameter for the region 109 (such as the distal most 10-15 cm), then have a step up in diameter of between 10-25% of the distal most diameter for the proximal region 107 of the working length. The step up would occur over a tapered transition section between 3 and 10 mm in length, depending on the size of the step up and the need to make a smooth transition. Alternately, the catheter is used with a stepped sheath with a larger diameter proximal region. In this case, the catheter may be stepped up a length and diameter to match the stepped sheath. For example, if the sheath has a portion with larger diameter for the proximal 20 cm of the sheath, the catheter would have a larger diameter for the proximal 25 cm to allow for additional length through proximal adaptors and valves such as an RHV. The remaining distal region would have a smaller diameter, with a step up over a tapered transition section between 3 and 10 mm in length, depending on the size of the step up and the need to make a smooth transition.

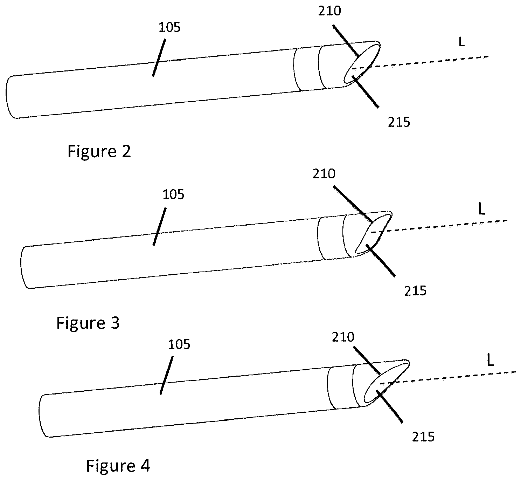

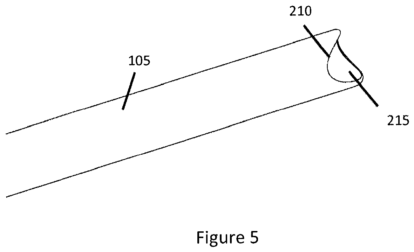

In some instances, a neurovascular catheter is used to aspirate clot or other obstruction in a cerebral or intracranial vessel. Figures 2-5 show examples of catheters having angled distal tips or distal edges. With reference to Figure 2, the distal region of a catheter 105 is shown. The catheter 105 has a distal-most tip or edge 210 that forms an opening 215 at the distal end of the catheter 105. The distal edge 210 forms an angle that is non-perpendicular relative to the longitudinal axis L. Such a tip defines a different sized opening 215 than if the tip were perpendicular to the axis L. That is, the opening 215 is larger and presents a larger suction area relative to a distal tip that is cut normal to the longitudinal axis. The catheter therefore may provide a larger suction force on the occlusion located near the tip. The larger area opening 215 also facilitates suctioning the clot into the lumen of the catheter, rather than just capturing the clot at the tip with suction force and pulling back the captured clot with the catheter. In Figure 2, the catheter 105 has an angled, straight edge 210 creating an elliptical opening 215. In Figures 3, 4 and 5, the distal edge 210 is curved or non-straight such that the distal opening 215 is non-planar and may offer greater opening without extending the tip length out as much, which may optimize the contact area with the occlusion further. The distal edge 210 may be straight, curved, undulating, or irregular. In an embodiment with a cut hypotube-reinforced catheter, the distal tip of the hypotube can be formed with the non-square shape. In an embodiment with a radiopaque marker band, the radiopaque marker band may have a non-square edge which can then be used to create the non-square catheter tip shape. In an embodiment, the catheter may have an angled distal tip. That is, the distal tip of the catheter is angled or non-perpendicular relative to a longitudinal axis of the catheter.

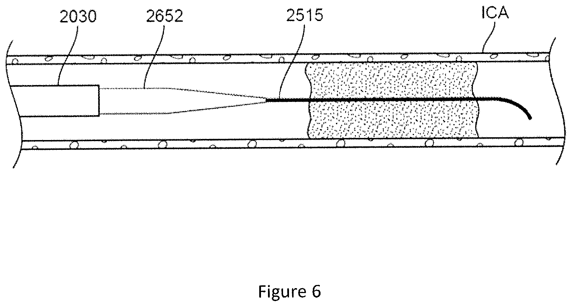

A cause of difficulty in advancing catheters through severe bends and across side branches is the mismatch between the catheter and the inner guiding components such as smaller catheters, microcatheters, or guidewires. One technique for advancing a catheter is called a tri-axial technique in which a smaller catheter or microcatheter is placed between the catheter and the guide wire. However, with current systems the smaller catheter has a diameter mismatch between either the larger catheter, the guide wire, or both, which creates a step in the system's leading edge as the system is advanced in the vasculature. This step may cause difficulty when navigating very curved vessels, especially at a location where there is a side-branch, for example the ophthalmic artery. In an embodiment, as shown in Figure 6, the catheter 105 is supplied with a tapered co-axial inner member 2652 that replaces the smaller catheter generally used. The inner member 2652 is sized and shaped to be inserted through the internal lumen of the catheter. The inner member 2652 has a tapered region with an outer diameter that forms a smooth transition between the inner diameter of the catheter 2030 and the outer diameter of a guidewire 2515 or microcatheter that extends through an internal lumen of the inner member 2652. In an embodiment, the tapered dilator or inner member 2652, when positioned within the catheter, creates a smooth transition between the distal-most tip of the larger catheter 105 and the outer diameter of a guide wire 2515 which may be in the range of 0.014" and 0.018" diameter for example. For example, the inner luminal diameter may be between .020" and .024". 1 inch = 2.54 cm. In another embodiment, the inner diameter is configured to accept a microcatheter with an outer diameter in the range of .030" to .040" or an .035" guide wire in the inner lumen, for example the inner luminal diameter may be .042" to .044".

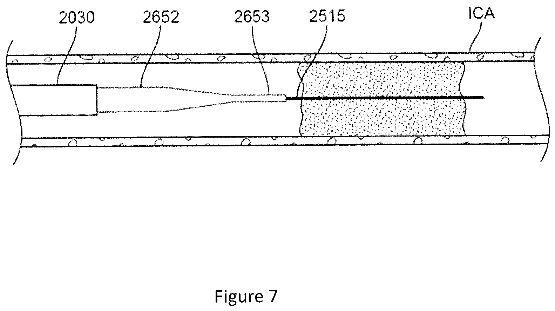

In a variation of this embodiment, shown in Figure 7, in addition to the tapered region, the inner member 2652 includes an extension formed of a uniform diameter or a single diameter, distal-most region 2653 that extends distally past the tapered portion of the inner member 2652. In this embodiment the distal region 2653 of the inner member 2652 may perform some or all of the functions that a microcatheter would do during an interventional procedure, for example cross an occlusion to perform distal angiograms, inject intraarterial agents, or deliver devices such as aneurysm coils or stent retrievers. In this manner, a microcatheter would not need to be exchanged for the dilator for these steps to occur.

The material of the dilator (inner member 2652) is flexible enough and the taper is long enough to create a smooth transition between the flexibility of the guide wire and the catheter. This configuration will facilitate advancement of the catheter through the curved anatomy and into the target cerebral vasculature. In an embodiment, the dilator is constructed to have variable stiffness, for example the distal most section is made from softer material, with increasingly harder materials towards the more proximal sections. In an embodiment, distal end of the tapered dilator has a radiopaque marker such as a platinum/iridium band, a tungsten, platinum, or tantalum-impregnated polymer, or other radiopaque marker.

Although embodiments of various example methods and devices are described herein in detail with reference to certain versions, it should be appreciated that other versions, embodiments, example methods of use, and combinations thereof are also possible. The invention is defined by the following claims.

## Claims

1. An interventional catheter (105) for treating an artery, comprising:
an elongated body sized and shaped to be transcervically introduced into a common carotid artery at an access location in the neck, the elongated body sized and shaped to be navigated distally to an intracranial artery through the common carotid artery via the access location in the neck; and
an internal lumen in the elongated body, the internal lumen forming a proximal opening in a proximal region of the elongated body and a distal opening (215) in a distal region (109) of the elongated body;
wherein the elongated body has a proximal most section and a distal most section wherein the proximal most section is a stiffest portion of the elongated body, and wherein the elongated body has an overall length and a distal most section length such that the distal most section can be positioned in an intracranial artery and while simultaneously at least a portion of the proximal most section is positioned in the common carotid artery during use,
wherein the distal most section of the catheter (105) has an inner diameter of 0.889mm to 1.574mm (.035 to .062 inch),
wherein the catheter (105) can navigate around a 180° bend with a radius of 2.54mm (.100") without kinking; and
the catheter (105) is configured to resist collapse when connected to a vacuum up to 84.660 kPa (25 inHg) while navigating around a 180° bend with a radius of 2.54mm (.100"),
wherein the catheter has a Polytetrafluoroethylene (PTFE) inner liner having a thickness of 0.0127mm to 0.0254mm (0.0005 inch to 0.0010 inch).

2. A catheter (105) as in claim 1, wherein the elongated body includes a first transition section between the proximal section and the distal most section, and wherein the transition section has a stiffness between a stiffness of the proximal most section and the distal most section.

3. A catheter (105) as in claim 1, wherein the elongated body has a working length and wherein the distal most section is between 3% and 15% of the length of the working length of the elongated body.

4. A catheter (105) as in claim 1, wherein the elongated body has a working length and wherein the distal most section is between 4% and 25% of the length of the working length of the elongated body.

5. A catheter (105) as in claim 2, wherein the first transition section is between 7 and 35% of the length of the working length of the catheter (105), or preferably
wherein the first transition section is between 6 and 20% of the length of the working length of the catheter (105).

6. A catheter (105) as in claim 3, wherein the elongated body has an inner diameter of either 1.727mm to 2.413mm (.068" to .095") and a working length of 40-60 cm, or 0.889mm to 1.574mm (.035" to .062") and a working length of 50-70 cm.

7. A catheter (105) as in claim 1, wherein the elongated body varies in stiffness moving along at least a portion of the length of catheter (105).

8. A catheter (105) as in claim 7, wherein the variation in stiffness is represented by smooth change in flexibility without any sudden changes in flexibility.

9. A catheter (105) as in claim 7, wherein the flexibility of the elongated body transitions gradually moving along its length without any discrete variations in flexibility from one section of the elongated body to an adjacent section of the elongated body.

10. A catheter (105) as in claim 1, wherein the proximal most section has a stiffness in the range of 100 to 500 N-mm².

11. A catheter (105) as in claim 1, wherein the distal most section has a stiffness in the range of 3 to 10 N-mm².

12. A catheter (105) as in claim 1, wherein the distal most section is between 5 and 15 cm in length, or preferably
wherein the distal most section is between 3 and 10 cm in length.

13. A catheter (105) as in claim 2, wherein the first transition section is 5 and 10 cm in length, or preferably
wherein the first transition section is 4 and 8 cm in length.

14. A catheter (105) as in claim 1, wherein the elongated body has a working length of 40 cm and the proximal most section has a length of 10 to 30 cm.

## Patentansprüche

1. Ein Interventionskatheter (105) zur Behandlung einer Arterie, das Folgendes umfasst:
einen länglichen Körper, der so bemessen und geformt ist, dass er transzervikal in eine gemeinsame Karotide an einer Zugangsstelle im Hals eingeführt werden kann, wobei der längliche Körper so bemessen und geformt ist, dass er distal zu einer intrakraniellen Arterie durch die gemeinsame Karotide über die Zugangsstelle im Hals navigiert werden kann; und
ein inneres Lumen in dem länglichen Körper, wobei das innere Lumen eine proximale Öffnung in einem proximalen Bereich des länglichen Körpers und eine distale Öffnung (215) in einem distalen Bereich (109) des länglichen Körpers bildet;
wobei der längliche Körper einen proximalsten Abschnitt und einen distalsten Abschnitt aufweist, wobei der proximalste Abschnitt ein steifster Teil des länglichen Körpers ist, und wobei der längliche Körper eine Gesamtlänge und eine distalste Abschnittslänge aufweist, so dass der distalste Abschnitt in einer intrakraniellen Arterie positioniert werden kann, während gleichzeitig mindestens ein Teil des proximalsten Abschnitts während der Verwendung in der gemeinsamen Karotide positioniert ist,
wobei der distalste Abschnitt des Katheters (105) einen Innendurchmesser von 0,889 mm bis 1,574 mm (0,035 bis 0,062 Zoll) aufweist,
wobei der Katheter (105) um eine 180°-Biegung mit einem Radius von 2,54 mm (100") ohne Knicken navigieren kann; und
der Katheter (105) so konfiguriert ist, dass er einem Kollaps widersteht, wenn er an ein Vakuum von bis zu 84,660 kPa
(25 inHg) angeschlossen ist, während er um eine 180°-Biegung mit einem Radius von 2,54 mm (100") navigiert, wobei der Katheter eine Innenauskleidung aus Polytetrafluorethylen (PTFE) mit einer Dicke von 0,0127 mm bis 0,0254 mm (0,0005 Zoll bis 0,0010 Zoll) aufweist.

2. Katheter (105) nach Anspruch 1, wobei der längliche Körper einen ersten Übergangsabschnitt zwischen dem proximalsten Abschnitt und dem distalsten Abschnitt aufweist, und wobei der Übergangsabschnitt eine Steifigkeit zwischen einer Steifigkeit des proximalsten Abschnitts und des distalsten Abschnitts aufweist.

3. Katheter (105) nach Anspruch 1, wobei der längliche Körper eine Arbeitslänge aufweist und wobei der distalste Abschnitt zwischen 3 % und 15 % der Länge der Arbeitslänge des länglichen Körpers beträgt.

4. Katheter (105) nach Anspruch 1, wobei der längliche Körper eine Arbeitslänge aufweist und wobei der distalste Abschnitt zwischen 4 % und 25 % der Länge der Arbeitslänge des länglichen Körpers beträgt.

5. Katheter (105) nach Anspruch 2, wobei der erste Übergangsabschnitt zwischen 7 und 35 % der Länge der Arbeitslänge des Katheters (105) beträgt, oder vorzugsweise
wobei der erste Übergangsabschnitt zwischen 6 und 20 % der Länge der Arbeitslänge des Katheters (105) beträgt.

6. Katheter (105) nach Anspruch 3, wobei der längliche Körper einen Innendurchmesser von entweder 1,727 mm bis 2,413 mm (0,068" bis 0,095") und eine Arbeitslänge von 40-60 cm oder 0,889 mm bis 1,574 mm (0,035" bis 0,062") und eine Arbeitslänge von 50-70 cm aufweist.

7. Katheter (105) nach Anspruch 1, wobei der längliche Körper in seiner Steifigkeit variiert, wenn er sich entlang mindestens eines Teils der Länge des Katheters (105) bewegt.

8. Katheter (105) nach Anspruch 7, wobei die Veränderung der Steifigkeit durch eine gleichmäßige Veränderung der Flexibilität ohne plötzliche Veränderungen der Flexibilität dargestellt wird.

9. Katheter (105) nach Anspruch 7, wobei die Flexibilität des länglichen Körpers allmählich übergeht, indem er sich entlang seiner Länge bewegt, ohne irgendwelche diskreten Variationen der Flexibilität von einem Abschnitt des länglichen Körpers zu einem benachbarten Abschnitt des länglichen Körpers.

10. Katheter (105) nach Anspruch 1, wobei der proximalste Abschnitt eine Steifigkeit im Bereich von 100 bis 500 N-mm² aufweist.

11. Katheter (105) nach Anspruch 1, wobei der distalste Abschnitt eine Steifigkeit im Bereich von 3 bis 10 N-mm² aufweist.

12. Katheter (105) nach Anspruch 1, wobei der distalste Abschnitt zwischen 5 und 15 cm lang ist, oder vorzugsweise
wobei der distalste Abschnitt eine Länge zwischen 3 und 10 cm aufweist.

13. Katheter (105) nach Anspruch 2, wobei der erste Übergangsabschnitt eine Länge von 5 und 10 cm aufweist, oder vorzugsweise
wobei der erste Übergangsabschnitt zwischen 4 und 8 cm lang ist.

14. Katheter (105) nach Anspruch 1, wobei der längliche Körper eine Arbeitslänge von 40 cm hat und der proximalste Abschnitt eine Länge von 10 bis 30 cm hat.

## Revendications

1. Cathéter interventionnel (105) pour traiter une artère, comprenant :
un corps allongé dimensionné et façonné pour être introduit de manière transcervicale dans une artère carotide commune au niveau d'un emplacement d'accès dans le cou, le corps allongé étant dimensionné et façonné pour être guidé de manière distale jusqu'à une artère intracrânienne à travers l'artère carotide commune via l'emplacement d'accès dans le cou ; et
une lumière interne dans le corps allongé, la lumière interne formant une ouverture proximale dans une région proximale du corps allongé et une ouverture distale (215) dans une région distale (109) du corps allongé ;
dans lequel le corps allongé présente une section la plus proximale et une section la plus distale, dans lequel la section la plus proximale est une partie la plus rigide du corps allongé, et dans lequel le corps allongé présente une longueur totale et une longueur de section la plus distale de sorte que la section la plus distale peut être positionnée dans une artère intracrânienne et tandis que simultanément, au moins une partie de la section la plus proximale est positionnée dans l'artère carotide commune pendant l'utilisation,
dans lequel la section la plus distale du cathéter (105) présente un diamètre intérieur de 0,889 mm à 1,574 mm (0,035 à 0,062 pouce),
dans lequel le cathéter (105) peut naviguer autour d'une courbure de 180° avec un rayon de 2,54 mm (0,100 po) sans se plier ; et
le cathéter (105) est configuré pour résister à l'effondrement lorsqu'il est connecté à un vide allant jusqu'à 84,660 kPa (25 inHg) lors de la navigation dans une courbure de 180° avec un rayon de 2,54 mm (0,100 po),
dans lequel le cathéter présente un revêtement intérieur en polytétrafluoroéthylène (PTFE) présentant une épaisseur de 0,0127 mm à 0,0254 mm (0,0005 pouce à 0,0010 pouce).

2. Cathéter (105) selon la revendication 1, dans lequel le corps allongé comporte une première section de transition entre la section proximale et la section la plus distale, et dans lequel la section de transition présente une rigidité entre une rigidité de la section la plus proximale et la section la plus distale.

3. Cathéter (105) selon la revendication 1, dans lequel le corps allongé présente une longueur utile et dans lequel la section la plus distale est comprise entre 3 % et 15 % de la longueur de la longueur utile du corps allongé.

4. Cathéter (105) selon la revendication 1, dans lequel le corps allongé présente une longueur utile et dans lequel la section la plus distale est comprise entre 4 % et 25 % de la longueur de la longueur utile du corps allongé.

5. Cathéter (105) selon la revendication 2, dans lequel la première section de transition est comprise entre 7 et 35 % de la longueur de la longueur utile du cathéter (105), ou de préférence
dans lequel la première section de transition est comprise entre 6 et 20 % de la longueur de la longueur utile du cathéter (105).

6. Cathéter (105) selon la revendication 3, dans lequel le corps allongé présente un diamètre intérieur soit de 1,727 mm à 2,413 mm (0,068 po à 0,095 po) et une longueur utile de 40 à 60 cm, ou de 0,889 mm à 1,574 mm (0,035 po à 0,062 po) et une longueur utile de 50 à 70 cm.

7. Cathéter (105) selon la revendication 1, dans lequel le corps allongé varie en rigidité en se déplaçant le long d'au moins une partie de la longueur du cathéter (105) .

8. Cathéter (105) selon la revendication 7, dans lequel la variation de rigidité est représentée par un changement doux de flexibilité sans aucun changement brusque de flexibilité.

9. Cathéter (105) selon la revendication 7, dans lequel la flexibilité du corps allongé se déplace progressivement sur sa longueur sans aucune variation discrète de flexibilité d'une section du corps allongé à une section adjacente du corps allongé.

10. Cathéter (105) selon la revendication 1, dans lequel la section la plus proximale présente une rigidité comprise dans la plage allant de 100 à 500 N-mm².

11. Cathéter (105) selon la revendication 1, dans lequel la section la plus distale présente une rigidité comprise dans la plage allant de 3 à 10 N-mm².

12. Cathéter (105) selon la revendication 1, dans lequel la section la plus distale présente une longueur comprise entre 5 et 15 cm, ou de préférence
dans lequel la section la plus distale présente une longueur comprise entre 3 et 10 cm.

13. Cathéter (105) selon la revendication 2, dans lequel la première section de transition présente une longueur de 5 et 10 cm, ou de préférence
dans lequel la première section de transition présente une longueur de 4 et 8 cm.

14. Cathéter (105) selon la revendication 1, dans lequel le corps allongé présente une longueur utile de 40 cm et la section la plus proximale présente une longueur de 10 à 30 cm.
